# FASCICULE DE BREVET EUROPEEN

(11) **EP 0 935 459 B1**
(45) Date de publication et mention de la délivrance du brevet: **12.06.2002**
(21) Numéro de dépôt: 97943017.0
(22) Date de dépôt: 29.09.1997
(51) Int. Cl.: A61K 9/16, A61K 9/50

(54) **PROCEDE DE FABRICATION D'UNE COMPOSITION PHARMACEUTIQUE A LIBERATION MODIFIEE DE PRINCIPE ACTIF, COMPORTANT UNE MATRICE**
VERFAHREN ZUR HERSTELLUNG EINER PHARMAZEUTISCHEN ZUSAMMENSETZUNG MIT VERÄNDERTER WIRKSTOFFSFREISETZUNG, ENTHALTEND EINE MATRIX
METHOD FOR PREPARING A PHARMACEUTICAL COMPOSITION WITH MODIFIED RELEASE OF THE ACTIVE PRINCIPLE, COMPRISING A MATRIX

(30) Priorité: 01.10.1996 FR 9612156
(43) Date de publication de la demande: 18.08.1999
(73) Titulaire: GATTEFOSSE S.A., 69800 Saint Priest (FR)
(72) Inventeur: FARAH, Nabil, F-69007 Lyon (FR); BARTHELEMY, Philippe, F-69780 Mions (FR); JOACHIM, Joseph, F-13010 Marseille (FR)
(74) Mandataire: Vuillermoz, Bruno
(86) Numéro de dépôt international: FR9701710
(87) Numéro de publication internationale: WO9814176

(56) Documents cités:
- EP-A- 0 421 581
- WO-A-94/12180
- WO-A-94/27557
- FR-A- 2 573 307
- US-A- 4 129 666
- PATENT ABSTRACTS OF JAPAN vol. 017, no. 195 (C-1049), 16 avril 1993 & JP 04 342523 A (MITSUBISHI KASEI CORP), 30 novembre 1992, & DATABASE WPI Section Ch, Week 932 Derwent Publications Ltd., London, GB; Class B07, AN 93-014043 & JP 04 342 523 (MITSUBISHI KASEI CORP) , 30 novembre 1992
- ACHANTE A.S. ET AL: "Development of hot melt coating methods" DRUG DEV. IND. PHARM., vol. 23, no. 5, 1997, pages 441-449, XP002051820
- THOMSEN L. ET AL: "Prolonged release matrix pellets prepared by melt pelletization. II. Hydrophobic substances as meltable binders" DRUG DEV. IND. PHARM., vol. 20, no. 7, 1994, pages 1179-1197, XP000677105
- JOZWIAKOWSKI M.J. ET AL: "Characterization of a hot-melt fluid bed coating process for fine granules" PHARM. RES., vol. 7, no. 11, 1990, pages 1119-1126, XP002052859

## Description

L'invention concerne un procédé de fabrication d'une composition pharmaceutique à libération modifiée de principe actif, comportant une matrice.

Par « composition pharmaceutique à libération modifiée de principe actif», on désigne les compositions pharmaceutiques à libération accélérée, prolongée et différée de principe actif.

Il existe différents types de compositions pharmaceutiques à libération modifiée de principe actif. On utilise notamment des compositions comprenant d'une part, des granulés non enrobés constituant la dose de principe actif immédiatement disponible et d'autre part, des granulés enrobés assurant la libération modifiée de principe actif.

On utilise également des compositions comportant un effet matriciel.

L'invention se rapporte plus particulièrement au procédé de fabrication de ces dernières.

Dans ce type de composition, le principe actif est dispersé ou enrobés dans un système solide, appelé matrice. La libération du principe actif hors de la matrice est effectuée par contact des liquides biologiques avec ladite matrice. Plus précisément, les liquides biologiques migrent à travers la matrice et solubilisent les principes actifs et ceux-ci sont libérés par diffusion à travers la matrice qui, simultanément, module le flux de libération.

On distingue les matrices hydrophiles et les matrices hydrophobes.

Dans les compositions comportant une matrice hydrophile, la matrice est constituée d'un polymère hydrophile insoluble dont la concentration est comprise entre 25 % et plus de 50 % du poids de la composition, donc élevée. Ce polymère est choisi parmi les esters de cellulose, les esters carboxyvinyliques, ou encore les esters acryliques ou méthacryliques. Au contact des liquides biologiques, la matrice s'hydrate et gonfle, formant un réseau très dense de polymères à travers lesquels diffusent les principes actifs solubles. Par ailleurs, on peut être amené à ajouter des produits lipidiques dans la composition, notamment des esters de glycérol, comme illustré par exemple dans le document FR-B-2 573 307, et ce, pour moduler le gonflement matriciel. En outre, ces compositions renferment de nombreux adjuvants, souvent onéreux, à des concentrations élevées, ce qui augmente considérablement le coût de la composition.

Ces compositions sont obtenues par granulation puis compression du mélange formés du polymère, des principes actifs et des divers adjuvants. Ces techniques font souvent appel à l'emploi de solvants organiques, qu'il est ensuite indispensable de récupérer, pour éviter leur dispersion dans l'atmosphère. En outre, il peut subsister dans le produit final des traces de solvant toxiques, lesquelles traces doivent être nécessairement quantifiées.

En d'autres termes, la préparation de ces compositions conduit à un coût de production élevé, dû au coût des différents constituants de la composition, à leurs proportions élevées et aux contraintes techniques à surmonter.

Dans les compositions comportant une matrice hydrophobe, la matrice est constituée d'un agent matriciel lipidique, d'origine naturelle, par exemple les cires d'abeilles, possédant une innocuité élevée, mais dont la composition d'un lot à l'autre varie et dont la stabilisation dans le temps reste peu satisfaisante.

Comme précédemment, ces compositions sont généralement obtenues par granulation, par voie humide ou solvant; puis compression, faisant appel à des proportions élevées de chacun des constituants.

Le document WO 94/12180 décrit un procédé d'enrobage de granules de cimétidine par pulvérisation d'une huile végétale hydrogénée liquéfiée à raison de 23% en poids de la composition enrobée. Aucune indication n'est donnée concernant le profil de libération obtenu.

Le document EP-A-0 421 581 décrit un procédé d'enrobage de principe actif par deux couches successives, respectivement une première couche à base d'éthyl cellulose et une seconde couche constituée de cire de carnauba, de tristéarate de glycérol, de paraffine et de polyvinyle pyrrolidone K. L'enrobage de la seconde couche est effectué dans des proportions très élevées (voir exemple 2).

Le but de l'invention est donc de proposer un nouveau procédé de fabrication d'une composition pharmaceutique à libération modifiée de principe actif en ayant pour objectif de diminuer de manière significative le nombre et les proportions de chacun des constituants de même que le nombre d'opérations, permettant ainsi d'obtenir une formulation simple à mettre en oeuvre, et de faible coût et reproductible.

Dans une première forme de réalisation, l'invention concerne le procédé objet de la revendication 1.

En d'autres termes, l'invention réside dans la mise en oeuvre d'un procédé spécifique qui permet de diminuer le nombre d'adjuvants nécessaire à la réalisation de la composition, et donc d'aboutir à une formule extrêmement simple et de faible coût.

En outre, le procédé de l'invention ne nécessite pas de phase d'évaporation, ni de phase de séchage, puisqu'il ne requiert pas d'étape de granulation par voie humide ou solvant, permettant ainsi de s'affranchir de tout risque dû à la présence de résidus toxiques dans le produit final. De plus, il n'est plus besoin d'effectuer le dosage des traces de solvants, analyse très coûteuse.

Selon le procédé de l'invention, on peut modifier les conditions de pulvérisation et donc les caractéristiques d'enrobage pour jouer sur le profil de libération du principe actif, en faisant varier plusieurs paramètres, dont les caractéristiques de réglage restent simples.

Ainsi, on peut augmenter la pression d'air de pulvérisation et diminuer simultanément le débit de pulvérisation de l'agent matriciel lipidique afin de favoriser la formation d'un film homogène d'agent matriciel lipidique autour des grains.

On obtient dans ce cas un profil de libération de principe actif, c'est-à-dire un pourcentage de dissolution en fonction du temps très bas, correspondant à une libération lente du principe actif.

Dans une seconde forme de réalisation, l'invention concerne le procédé objet de la revendication 4.

Pour favoriser l'agglomération des grains entre eux, on diminue la pression d'air de pulvérisation en augmentant simultanément le débit de pulvérisation de l'agent matriciel lipidique.

On obtient dans ce cas un profil de libération des grains obtenus très élevé, correspondant à une libération rapide du principe actif.

En pratique, et en fonction de la masse de poudre mise en oeuvre, la valeur du débit de pulvérisation de l'agent matriciel lipidique est de deux à quatre fois plus élevé lorsqu'on désire favoriser l'agglomération des grains entre eux que lorsqu'on désire favoriser la formation d'un film homogène autour des grains.

En revanche, la valeur de la pression d'air de pulvérisation est de un à deux fois plus faible lorsqu'on désire favoriser l'agglomération des grains entre eux que lorsqu'on désire favoriser la formation d'un film homogène autour des grains.

Selon le procédé de l'invention, il est possible, après avoir déterminé un profil de libération de principe actif donné, de faire varier les valeurs de pression d'air de pulvérisation et de débit de pulvérisation tout au long de l'étape d'enrobage, permettant de favoriser la formation d'un film homogène autour des grains ou de favoriser l'agglomération des grains.

Une fois la séquence de la durée de la pression d'air de pulvérisation et du débit de pulvérisation déterminée, l'opération d'enrobage peut être effectuée en continu et de façon automatisée.

Selon une autre caractéristique de l'invention, la température du mélange d'agent matriciel liquéfié et d'air de pulvérisation doit être supérieure de 35° C à 60° C à la température de fusion de l'agent matriciel lipidique.

De même, la température de l'air de fluidisation et celle de la poudre doit être égale à la température de fusion de l'agent matriciel lipidique, plus ou moins 10°C.

Par ailleurs pour obtenir un mélange de grains individualisés, on utilise un appareil à lit d'air fluidisé ou un appareil à turbine.

De plus, la pulvérisation de l'agent matriciel lipidique peut être effectuée par la technique dite « AIR SPRAY », c'est-à-dire une pulvérisation liquide sous pression en présence d'air comprimé.

Selon une première forme de réalisation, on utilise une poudre comprenant le principe actif et l'adjuvant. En d'autres termes, après mélange et fluidisation de l'ensemble des constituants de la poudre, on pulvérise l'agent matriciel lipidique sur les grains individualisés obtenus.

Lorsqu'on désire conditionner le produit obtenu sous forme de sachet ou de gélule, on règle la pression d'air de pulvérisation et le débit de pulvérisation d'agent matriciel lipidique à une valeur permettant de favoriser la formation d'un film homogène d'agent matriciel lipidique autour des grains.

Lorsqu'on désire obtenir des comprimés, les grains enrobés sont soumis à une étape de compression.

De façon tout à fait surprenante, on observe que dans le cas où on enrobe les grains individualisés tout en favorisant la formation d'un film homogène autour desdits grains, alors qu'ils présentent un profil de libération très bas avant compression, ceux-ci présentent au contraire un profil de libération élevé après compression.

A l'inverse, et de façon tout aussi surprenante, dans le cas où l'on favorise l'agglomération des grains individualisés, alors que lesdits grains présentent un profil de libération élevé avant compression, au contraire ils présentent un profil de libération bas après compression.

Comme déjà dit, il apparaît donc très avantageux de faire varier les conditions de pulvérisation tout au long de l'opération d'enrobage afin de favoriser plus ou moins la libération du principe actif.

Selon une autre forme de réalisation de l'invention, on utilise une poudre constituée exclusivement du principe actif.

Selon cette technique, les grains de principe actif enrobés sont mélangés à froid avec des adjuvants non enrobés.

De même, on peut utiliser une poudre constituée exclusivement d'adjuvan(s),

Dans ce cas, les grains d'adjuvants enrobés sont mélangés avec le principe actif non enrobés.

Comme précédemment, afin d'obtenir des comprimés, le mélange obtenu est soumis à une étape de compression.

Le mélange obtenu peut être directement conditionné sous forme de sachets ou gélules.

Pour éviter l'adhésion des grains enrobés obtenus, que ce soit dans le cas où l'ensemble des grains est traité, ou que ce soit dans le cas où une partie seulement des grains est traitée, on intercale entre l'étape d'enrobage et l'étape de mise en forme pharmaceutique, une étape de lubrification des grains.

Par ailleurs, pour obtenir une meilleure stabilité de la composition pharmaceutique, c'est-à-dire pour minimiser les modifications relatives à la libération du/des principes actifs dans le temps, les granulés ou comprimés obtenus peuvent être soumis à une étape de mûrissement dans une étuve, pendant au moins 8 heures, à une température comprise entre 45 et 60° C, avantageusement 55° C.

Pour résoudre le problème d'obtenir une composition dont les proportions de constituants sont faibles, on utilise une quantité d'agent matriciel représentant en poids de 1 à 15 % de la composition finale, avantageusement de 2 à 5 %.

Pour une valeur inférieure à 1% d'agent matriciel lipidique, on ne parvient pas à obtenir un enrobage régulier.

Pour une valeur supérieure à 15%, le procédé devient beaucoup moins intéressant économiquement.

Ces proportions sont donc très faibles par rapport à celles utilisées dans l'art antérieur, notamment dans le document précité FR-B-2 573 307, dans lequel les proportions décrites sont largement supérieures à 15 % en poids de la composition finale, en général 30%.

Selon un premier mode de réalisation de l'invention, on utilise, en tant qu'agent matriciel lipidique un ester d'acide béhénique et d'alcool.

Avantageusement, l'alcool est choisi dans le groupe comprenant le glycérol, le polyglycérol, le propylène glycol, le propylène glycol associé à l'oxyde d'éthylène et le polyéthylène glycol.

Ces agents matriciels, présentent l'avantage d'avoir un point de fusion supérieur à 50° C, ce qui évite leur désintégration à la température de compression. Par ailleurs, ce point de fusion est supérieur à la température interne du corps humain (37° C), ce qui permet à l'agent lipidique d'avoir un comportement matriciel plus marqué.

En outre, la pulvérisation d'un ester d'acide gras et d'alcool en tant qu'agent matriciel lipidique, permet outre le fait d'accélérer ou de ralentir la libération du principe actif, de masquer par ailleurs le goût de la matière première. Ceci présente un réel intérêt dans la mesure où aucune des techniques de masquage actuelles ne permet de masquer le goût des matières premières sans ralentir excessivement la libération du principe actif.

Avantageusement, on utilise l'ester d'acide béhénique et de glycérol présentant un point de fusion compris entre 69 et 74° C, donc bien supérieur à 50° C. Cet ester résulte de l'estérification directe de l'acide béhénique sur le glycérol pour aboutir à un mélange de mono-, di-, et tribéhénate de glycérol.

Selon un autre mode de réalisation, l'agent matriciel lipidique est un ester d'acide palmitostéarique et d'alcool.

Selon une autre caractéristique de l'invention, l'adjuvant est choisi parmi les agents diluants hydrophobes, les agents diluants hydrophiles, les agents liants, les agents lubrifiants, seuls ou en mélange.

Dans une forme avantageuse de réalisation, l'agent diluant hydrophobe est le phosphate dicalcique et l'agent diluant hydrophile est le lactose.

Le phosphate dicalcique présente l'avantage d'être d'un coût très faible, ce qui contribue à réduire le coût final de la composition.

Par ailleurs, l'utilisation du lactose, en tant qu'agent diluant hydrophile, permet d'ajuster l'équilibre hydrophile/lipophile nécessaire pour la libération de principe actif.

Pour favoriser la compressibilité des grains lors de la fabrication de comprimés, on utilise en temps qu'agent liant, le polyvinylpyrrolidone, ce qui permet de diminuer les forces de compression de la composition pharmaceutique.

Pour éviter l'adhésion de la poudre sur les parois de la machine lors de l'opération de compression, la composition pharmaceutique comprend un agent lubrifiant choisi dans le groupe comprenant le stéarate de magnésium et le talc siliconé, seuls ou en combinaison.

Avantageusement, le talc siliconé est composé de 80 % de talc et de 20 % d'huile de silicone.

L'invention concerne également la composition obtenue par le procédé précédemment décrit.

Les avantages qui découlent de l'invention ressortiront mieux des exemples de réalisation suivants.

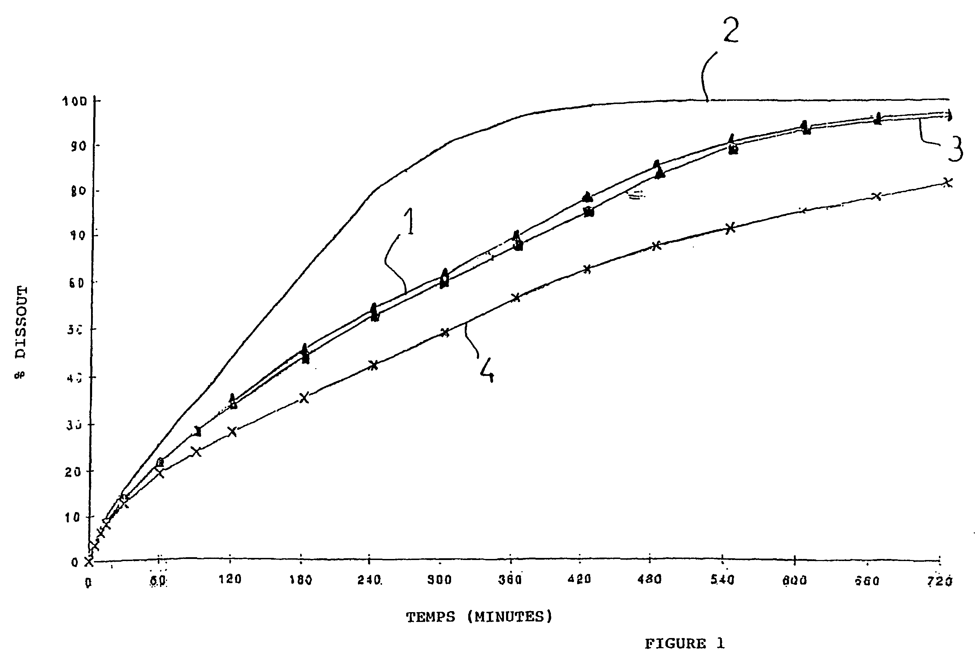

La figure 1 est une représentation en fonction du temps du profil de dissolution de lots de comprimés de théophylline préparés par le procédé de l'invention (lot 1, 3 et 4) ou par un procédé, lequel n'est pas couvert par les revendications (lot 2).

### Exemple 1

On prépare un mélange de 3 kg de poudre comprenant :
- principe actif : théophylline 1 920 g
- agent diluant hydrophobe : phosphate dicalcique dihydraté 90 g
- agent liant : polyvinylpyrrolidone 90 g

On prépare quatre lots de granulés par le procédé de l'invention, comprenant les étapes suivantes :
· on tamise le mélangé de poudre obtenu ;
· on mélange en chauffant ladite poudre au moyen d'un lit d'air fluidisé, afin d'obtenir des grains individualisés ;
- on liquéfie séparément l'agent matriciel lipidique (béhénate de glycérol commercialisé par le Demandeur sous la marque COMPRTTOL ® 880 ATO) à 120° Celsius ;
- on pulvérise l'agent matriciel lipidique sur le mélange de poudre chauffé,
- et enfin, on abaisse la température afin de permettre la solidification de l'agent matriciel lipidique.

On réalise ces étapes en faisant varier différents paramètres soit pour favoriser la formation d'un film homogène autour des grains, soit pour favoriser l'agglomération des grains conformément au tableau suivant :

| Paramètres | Lot 1 | Lot 2 | Lot 3 | Lot 4 |
|---|---|---|---|---|
| % en poids d'agent matriciel lipidique (COMPRITOL ® 888 ATO) | 5 | 4 | 4 | 5 |
| Débit d'air de fluidisation (m³/h) | 80 | 110 | 80 | 80 |
| Agglomération | | | | |
| Pression d'air d'atomisation (bar) | 2 | | 1,5 | 1,5 |
| Température du lit de poudre (°C) | 70 | | 70 | 74 |
| Débit de pulvérisation de COMPRITOL ® -(g/mn) | 42 | | 40 | 40 |
| Enrobage | | | | |
| Pression d'air d'atomisation (bar) | 2,5 | 3,5 | 2 | 2 |
| Température du lit de poudre (°C) | 70 | 66 | 71 | 70 |
| Débit de pulvérisation de COMPRITOL ® (g/mn) | 41 | 20 | 40 | 40 |

Les granulés ainsi obtenus sont mélangés dans un mélangeur avec un lubrifiant comprenant 1 % de stéréate de magnésium et 2 % de talc siliconé par rapport au poids de la préparation, pendant 10 minutes.

Pour obtenir du talc siliconé, on incorpore un taux de 20 % en poids d'huile de silicone (diméthicone fluide 100 CST de DOW CORNING) dans 80 % de talc en poids).

Sur la figure 1, on a représenté le profil de dissolution de lots de comprimés obtenus après une étape de compression des granulés, selon les paramètres du tableau précédent, comprenant 100 milligrammes de théophylline.

La courbe 1 correspond au lot 1.

La courbe 2 correspond au lot 2.

La courbe 3 correspond au lot 3.

La courbe 4 correspond au lot 4.

Ces courbes montrent que la libération de principe actif de la matrice sont fonction des paramètres de pression d'air de pulvérisation et de pulvérisation de l'agent matriciel lipidique.

Concernant le lot 1, on favorise d'abord l'agglomération des grains en maintenant la pression d'air de pulvérisation à 2 bars, puis on augmente cette pression à 2,5 bars afin de favoriser la formation d'un film homogène autour des grains.

Dans ce cas, on obtient après compression un profil de libération relativement élevé.

Concernant le lot 2, lequel ne fait pas partie de l'invention telle que définie dans les revendications, on favorise d'avantage la formation d'un film homogène d'agent matriciel lipidique autour des grains en fixant la pression d'air de pulvérisation à 3,5 bars et en diminuant le débit de pulvérisation.

On obtient de façon tout à fait surprenante après compression un profil de libération très élevé.

Concernant les lots 3 et 4, on ajuste les pressions d'air de pulvérisation à des valeurs permettant de favoriser l'agglomération des grains (1,5 bar) puis la formation du film homogène d'agent matriciel lipidique autour des grains (2 bars), et ce en continu au cours de l'étape de pulvérisation.

On observe qu'après compression et de façon très surprenante, on obtient des profils de libération très bas.

On constate par ailleurs qu'on obtient de très bons profils de libération et ce, avec des proportions d'agent matriciel très faibles de l'ordre de 4 à 5 % en poids de la composition finale.

Le procédé de fabrication de la composition de l'invention présente donc de nombreux avantages.

En effet, ce procédé se caractérise par le faible nombre de constituants qu'il met en oeuvre ainsi que les faibles proportions de chacun d'eux.

De plus, il permet de moduler le profil de libération de principe actif en faisant varier les conditions de débit de pulvérisation d'agent matriciel lipidique et de pression d'air de pulvérisation tout au long de l'étape d'enrobage, permettant ainsi de favoriser la formation d'un film homogène autour des grains et/ou l'agglomération des grains.

Il s'ensuit une économie importante dans le coût de production des compositions pharmaceutiques à libération modifiée.

## Revendications

1. Procédé de fabrication d'une composition pharmaceutique à libération modifiée de principe actif, comprenant au moins un principe actif, un agent matriciel lipidique constitué d'un ester d'alcool avec au moins un acide gras et au moins un adjuvant, **caractérisé en ce que**:
◆ tout en chauffant et en fluidisant, on mélange une poudre constituée d'au moins un composant sélectionné parmi le groupe comprenant, le principe actif et l'adjuvant, afin d'obtenir des grains individualisés ;
◆ séparément, on liquéfie, à chaud, ledit agent matriciel lipidique ;
◆ puis on enrobe à chaud ladite poudre, par pulvérisation de 1 à 15 % en poids de la composition finale dudit agent matriciel lipidique sur les grains individualisés, la pression d'air de pulvérisation, et le cas échéant le débit de pulvérisation, variant tout au long de l'étape d'enrobage, de sorte à favoriser, soit la formation d'un film homogène autour des grains, soit l'agglomération des grains entre eux ;
◆ enfin, on abaisse la température de l'ensemble afin de permettre la solidification de l'agent matriciel lipidique autour des grains.

2. Procédé de fabrication d'une composition pharmaceutique à libération modifiée selon la revendication 1, **caractérisé en ce qu'**on augmente tout au long de l'étape d'enrobage la pression d'air de pulvérisation, afin de favoriser la formation d'un film homogène d'agent matriciel lipidique autour des grains.

3. Procédé de fabrication d'une composition pharmaceutique à libération modifiée selon la revendication 2, **caractérisé en ce qu'**on diminue simultanément tout au long de l'étape d'enrobage le débit de pulvérisation de l'agent matriciel lipidique.

4. Procédé de fabrication d'une composition pharmaceutique à libération modifiée selon l'une des revendications 2 ou 3, **caractérisé en ce que** les grains enrobés sont soumis à une étape de compression, le comprimé obtenu présentant un profil de libération élevé de principe actif.

5. Procédé de fabrication d'une composition pharmaceutique à libération modifiée selon la revendication 1, **caractérisé en ce qu'**on diminue tout au long de l'étape d'enrobage la pression d'air de pulvérisation, afin de favoriser l'agglomération des grains.

6. Procédé de fabrication d'une composition pharmaceutique à libération modifiée selon la revendication 5, **caractérisé en ce qu'**on augmente tout au long de l'étape d'enrobage le débit de pulvérisation de l'agent matriciel lipidique.

7. Procédé de fabrication d'une composition pharmaceutique à libération modifiée selon l'une des revendications 5 ou 6, **caractérisé en ce que** les grains sont soumis à une étape de compression, le comprimé obtenu présentant un profil de libération bas de principe actif.

8. Procédé de fabrication d'une composition pharmaceutique à libération modifiée selon l'une des revendications 1 à 7, **caractérisé en ce que** la valeur du débit de pulvérisation de l'agent matriciel lipidique permettant de favoriser l'agglomération des grains est de deux à quatre fois plus élevée que celle permettant de favoriser la formation d'un film homogène d'agent matriciel lipidique autour des grains.

9. Procédé de fabrication selon l'une des revendications 1 à 8, **caractérisé en ce que** la valeur de la pression d'air de pulvérisation permettant de favoriser l'agglomération des grains est de un à deux fois plus faible que celle peimettant de favoriser la formation d'un film homogène d'agent matriciel lipidique autour des grains.

10. Procédé de fabrication selon l'une des revendications 1 à 9, **caractérisé :**
◆ **en ce que** la température du mélange d'agent matriciel lipidique liquéfié et d'air de pulvérisation est supérieur de 35° C à 60° C à la température de fusion dudit agent matriciel lipidique ;
◆ et **en ce que** la température du lit de poudre et de l'air de fluidisation est égale à la température de fusion de l'agent matriciel lipidique, plus ou moins 10°C.

11. Procédé de fabrication d'une composition pharmaceutique à libération modifiée selon l'une des revendications précédentes, **caractérisé en ce que** le mélange de grains individualisés est obtenu au moyen d'un lit d'air fluidisé.

12. Procédé de fabrication d'une composition pharmaceutique à libération modifiée selon la revendication 1, **caractérisé en ce qu'**on prépare une poudre comprenant le principe actif et l'adjuvant

13. Procédé de fabrication d'une composition pharmaceutique à libération modifiée selon la revendication 1, **caractérisé en ce qu'**on prépare une poudre constituée exclusivement du principe actif.

14. Procédé de fabrication d'une composition pharmaceutique à libération modifiée selon la revendication 1, **caractérisé en ce qu'**on prépare une poudre constituée exclusivement de l'adjuvant

15. Procédé de fabrication d'une composition pharmaceutique à libération modifiée selon la revendication 13, **caractérisé en ce qu'**on mélange à froid les grains enrobés de principe actif avec l'adjuvant non enrobé.

16. Procédé de fabrication d'une composition pharmaceutique à libération modifiée selon la revendication 14, **caractérisé en ce qu'**on mélange à froid les grains enrobés d'adjuvant avec le principe actif non enrobé.

17. Procédé de fabrication pharmaceutique à libération modifiée selon l'une des revendications 1 à 16, **caractérisé en ce qu'**on intercale entre l'étape permettant d'obtenir des grains enrobés et l'étape de mise en forme pharmaceutique une étape de lubrification des grains.

18. Procédé de fabrication d'une composition pharmaceutique à libération modifiée selon l'une des revendications 1 à 17, **caractérisé en ce qu'**on utilise une qasutité d'agent matriciel lipidique représentant en poids 2 à 5 % de la composition finale.

19. Procédé de fabrication d'une composition pharmaceutique à libération modifiée selon la revendication 18, **caractérisé en ce que** l'agent matriciel lipidique est un ester d'acide béhénique et d'alcool.

20. Procédé de fabrication d'une composition pharmaceutique à libération modifiée selon l'une des revendications 1 à 19, **caractérisé en ce qu'**on utilise un adjuvant choisi parmi les agents diluants hydrophobes, les agents diluants hydrophiles, les agents liants, les agents lubrifiants, seuls ou en mélange.

21. Procédé de fabrication d'une composition pharmaceutique à libération modifiée selon la revendication 20, **caractérisé en ce que** l'agent diluant hydrophobe est le phosphate dicalcique, et **en ce que** l'agent diluant hydrophile est le lactose.

22. Composition pharmaceutique à libération modifiée susceptible d'être obtenue par le procédé selon l'une des revendication 1 à 21.

## Claims

1. Process for the manufacture of a pharmaceutical composition with modified release of active principle comprising at least one active principle, a lipid matrix agent composed of partial esters of alcohol with at least one fatty acid and at least one adjuvant, **characterized in that**:
◆ a powder composed of at least one component, the active principle and the adjuvant, is mixed, while heating and fluidizing, in order to obtain individual grains;
◆ the said lipid matrix agent is liquefied separately under warm conditions;
◆ the said powder is then coated under warm conditions by spraying from 1 to 15% by weight of the final composition of the said lipid matrix agent over the individual grains, the spraying air pressure anf if necessary, the rate of spraying, varying along the coating step so as to promote either the formation of a homogeneous film around the grains either the agglomeration of the grains with one another;
◆ finally, the temperature of the combined product is lowered in order to allow the lipid matrix agent to solidify around the grains.

2. Process for the manufacture of a modified-release pharmaceutical composition according to Claim 1, **characterized in that** the spraying air pressure is increased along the coating step, in order to promote the formation of a homogeneous film of lipid matrix agent around the grains.

3. Process for the manufacture of a modified release pharmaceutical composition according to claim 2, **characterized in that** the rate of spraying of the lipid matrix agent is simultaneously decreased along the coating step.

4. Process for the manufacture of a modified-release pharmaceutical composition according to one of Claims 2 or 3, **characterized in that** the coated grains are subjected to a compression stage, the tablet obtained exhibiting a high release profile of active principle.

5. Process for the manufacture of a modified-release pharmaceutical composition according to claim 1 **characterized in that** the spraying air pressure is decreased along the coating step in order to promote the agglomeration of the grains.

6. Process for the manufacture of a modified-release pharmaceutical composition according to claim 5 **characterized in that** the rate of spraying of the lipid matrix agent is increased along the coating step.

7. Process for the manufacture of a modified-release pharmaceutical composition according to one of claims 5 or 6 **characterized in that** the coated grains are suspected to a compression stage, the tablet obtained exhibiting a low release profile of active principle.

8. Process for the manufacture of a modified-release pharmaceutical composition according to one of Claims 1 to 7, **characterized in that** the value of the rate of spraying of the lipid matrix agent which makes it possible to promote the agglomeration of the grains is two to four times higher than that which makes it possible to promote the formation of a homogeneous film of lipid matrix agent around the grains.

9. Process for the manufacture of a modified-release pharmaceutical composition according to one of Claims 1 to 8, **characterized in that** the value of the spraying air pressure which makes it possible to promote the agglomeration of the grains is one to two times lower than that which makes it possible to promote the formation of a homogeneous film of lipid matrix agent around the grains.

10. Process for the manufacture of a modified-release pharmaceutical composition according to one of Claims 1 to 9, **characterized:**
◆ **in that** the temperature of the mixture of liquefied lipid matrix agent and of spraying air is greater by 35°C to 60°C than the melting temperature of the said lipid matrix agent;
◆ and **in that** the temperature of the powder bed and of the fluidization air is equal to the melting temperature of the lipid matrix agent, plus or minus 10°C.

11. Process for the manufacture of a modified-release pharmaceutical composition according to one of the preceding claims, **characterized in that** the mixture of individual grains is obtained by means of an air-operated fluidized bed.

12. Process for the manufacture of a modified-release pharmaceutical composition according to Claim 1, **characterized in that** a powder comprising the active principle and the adjuvant is prepared.

13. Process for the manufacture of a modified-release pharmaceutical composition according to Claim 1, **characterized in that** a powder composed exclusively of the active principle is prepared.

14. Process for the manufacture of a modified-release pharmaceutical composition according to Claim 1, **characterized in that** a powder composed exclusively of the adjuvant is prepared.

15. Process for the manufacture of a modified-release pharmaceutical composition according to Claim 13, **characterized in that** the coated grains of active principle are mixed under cold conditions with the uncoated adjuvant.

16. Process for the manufacture of a modified-release pharmaceutical composition according to Claim 14, **characterized in that** the coated grains of adjuvant are mixed under cold conditions with the uncoated active principle.

17. Process for the manufacture of a modified-release pharmaceutical composition according to one of Claims 1 to 16, **characterized in that** a stage of lubrication of the grains is inserted between the stage which makes it possible to obtain coated grains and the stage of putting into a pharmaceutical form.

18. Process for the manufacture of a modified-release pharmaceutical composition according to one of Claims 1 to 17, **characterized in that** use is made of an amount of lipid matrix agent representing, by weight, 2 to 5% of the final composition.

19. Process for the manufacture of a modified-release pharmaceutical composition according to Claim 18, **characterized in that** the lipid matrix agent is an ester of behenic acid and of alcohol.

20. Process for the manufacture of a modified-release pharmaceutical composition according to one of Claims 1 to 19, **characterized in that** use is made of an adjuvant chosen from hydrophobic diluting agents, hydrophilic diluting agents, binding agents or lubricating agents, alone or as a mixture.

21. Process for the manufacture of a modified-release pharmaceutical composition according to Claim 20, **characterized in that** the hydrophobic diluting agent is dicalcium phosphate and **in that** the hydrophilic diluting agent is lactose.

22. A modified-release pharmaceutical composition obtainable according to the process of one claims 1 to 21.

## Patentansprüche

1. Verfahren zur Herstellung einer pharmazeutischen Zusammensetzung mit modifizierter Wirkstofffreisetzung, welches mindestens einen Wirkstoff, einen aus einem Alkoholester mit mindestens einer Fettsäure bestehenden fetthaltigen Matrixstoff und mindestens ein Adjuvans aufweist, **dadurch gekennzeichnet, daß**
• unter Aufheizung und Fluidisieren ein Pulver gemischt wird, welches mindestens aus einem Bestandteil besteht, das aus der Gruppe ausgewählt wird, die den Wirkstoff und das Adjuvans aufweist, um vereinzelte Körner zu erhalten;
• getrennt der genannte fetthaltige Matrixstoff heiß verflüssigt wird;
• anschließend das genannte Pulver heiß durch Aufsprühen von 1 bis 15 % des Gewichts der Endzusammensetzung des genannten fetthaltigen Matrixstoffes auf die vereinzelten Körnern umhüllt wird, wobei der Sprühluftdruck und gegebenenfalls die Sprühmenge während des gesamten Umhüllungsschrittes variiert wird, um entweder die Bildung eines homogenen Filmes um die Körner herum oder die Zusammenballung der Körner untereinander zu begünstigen;
• schließlich die Temperatur der Gesamtheit gesenkt wird, um die Verfestigung des fetthaltigen Matrixstoffes um die Körner zu erlauben.

2. Verfahren zur Herstellung einer pharmazeutischen Zusammensetzung mit modifizierter Wirkstofffreisetzung nach Anspruch 1, **dadurch gekennzeichnet, daß** während des gesamten Umhüllungsschrittes der Sprühluftdruck erhöht wird, um die Bildung eines homogenen Films des fetthaltigen Matrixstoffs um die Körner herum zu begünstigen.

3. Verfahren zur Herstellung einer pharmazeutischen Zusammensetzung mit modifizierter Wirkstofffreisetzung nach Anspruch 2, **dadurch gekennzeichnet, daß** simultan während des gesamten Umhüllungsschrittes die Sprühmenge des fetthaltigen Matrixstoffes gesenkt wird.

4. Verfahren zur Herstellung einer pharmazeutischen Zusammensetzung mit modifizierter Wirkstofffreisetzung nach einem der Ansprüche 2 oder 3, **dadurch gekennzeichnet, daß** die umhüllten Körner einem Kompressionsschritt unterzogen werden, wobei die erhaltene Tablette ein erhöhtes Wirkstofffreisetzungsprofil aufweist.

5. Verfahren zur Herstellung einer pharmazeutischen Zusammensetzung mit modifizierter Wirkstofffreisetzung nach Anspruch 1, **dadurch gekennzeichnet, daß** während des gesamten Umhüllungsschrittes der Sprühluftdruck gesenkt wird, um die Zusammenballung der Körner zu begünstigen.

6. Verfahren zur Herstellung einer pharmazeutischen Zusammensetzung mit modifizierter Wirkstofffreisetzung nach Anspruch 5, **dadurch gekennzeichnet, daß** während des gesamten Umhüllungsschrittes die Sprühmenge des fetthaltigen Matrixstoffes erhöht wird.

7. Verfahren zur Herstellung einer pharmazeutischen Zusammensetzung mit modifizierter Wirkstofffreisetzung nach einem der Ansprüche 5 oder 6, **dadurch gekennzeichnet, daß** die umhüllten Körner einem Kompressionsschritt unterzogen werden, wobei die erhaltene Tablette ein niedriges Wirkstofffreisetzungsprofil aufweist.

8. Verfahren zur Herstellung einer pharmazeutischen Zusammensetzung mit modifizierter Wirkstofffreisetzung nach einem der Ansprüche 1 bis 7, **dadurch gekennzeichnet, daß** der die Begünstigung der Zusammenballung der Körner ermöglichende Wert der Sprühmenge des fetthaltigen Matrixstoffs zwei bis viermal höher als der Wert ist, der die Begünstigung der Bildung eines homogenen Films des fetthaltigen Matrixstoffes um die Körner herum ermöglicht.

9. Herstellungsverfahren nach einem der Ansprüche 1 bis 8, **dadurch gekennzeichnet, daß** der die Begünstigung der Zusammenballung der Körnern ermöglichende Wert des Sprühluftdrucks ein bis zweimal niedriger als der Wert ist, der die Begünstigung der Bildung eines homogenen Films des fetthaltigen Matrixstoffes um die Körner herum ermöglicht.

10. Herstellungsverfahren nach einem der Ansprüche 1 bis 9, **dadurch gekennzeichnet,**
• **daß** die Temperatur der Mischung des verflüssigten fetthaltigen Matrixstoffes und der Sprühluft um 35°C bis 60°C über der Schmelztemperatur des genannten fetthaltigen Matrixstoffes liegt;
• und **daß** die Temperatur des Pulverbettes und der Fluidisierungsluft gleich der Schmelztemperatur des fetthaltigen Matrixstoffes +/- 10°C ist.

11. Verfahren zur Herstellung einer pharmazeutischen Zusammensetzung mit modifizierter Wirkstofffreisetzung nach einem der vorangehenden Ansprüche, **dadurch gekennzeichnet, daß** die Mischung der vereinzelten Körner mit Hilfe eines Wirbelluftbettes erfolgt.

12. Verfahren zur Herstellung einer pharmazeutischen Zusammensetzung mit modifizierter Wirkstofffreisetzung nach Anspruch 1, **dadurch gekennzeichnet, daß** ein Pulver, welches den Wirkstoff und das Adjuvans aufweist, zubereitet wird.

13. Verfahren zur Herstellung einer pharmazeutischen Zusammensetzung mit modifizierter Wirkstofffreisetzung nach Anspruch 1, **dadurch gekennzeichnet, daß** ein Pulver, welches nur den Wirkstoff aufweist, zubereitet wird.

14. Verfahren zur Herstellung einer pharmazeutischen Zusammensetzung mit modifizierter Wirkstofffreisetzung nach Anspruch 1, **dadurch gekennzeichnet, daß** ein Pulver, welches nur das Adjuvans aufweist, zubereitet wird.

15. Verfahren zur Herstellung einer pharmazeutischen Zusammensetzung mit modifizierter Wirkstofffreisetzung nach Anspruch 13, **dadurch gekennzeichnet, daß** die umhüllten Wirkstoff-Körner kalt mit dem nicht umhüllten Adjuvans gemischt werden.

16. Verfahren zur Herstellung einer pharmazeutischen Zusammensetzung mit modifizierter Wirkstofffreisetzung nach Anspruch 14, **dadurch gekennzeichnet, daß** die umhüllten Ajuvans-Körner kalt mit dem nicht umhüllten Wirkstoff gemischt werden.

17. Verfahren zur Herstellung einer pharmazeutischen Zusammensetzung mit modifizierter Wirkstofffreisetzung nach einem der Ansprüche 1 bis 16, **dadurch gekennzeichnet, daß** zwischen dem Schritt, der die Gewinnung umhüllter Körner ermöglicht, und dem Schritt der pharmazeutischen Formgebung ein Schritt der Trennmittelapplikation auf die Körner eingefügt wird.

18. Verfahren zur Herstellung einer pharmazeutischen Zusammensetzung mit modifizierter Wirkstofffreisetzung nach einem der Ansprüche 1 bis 17, **dadurch gekennzeichnet, daß** eine Menge des fetthaltigem Matrixstoff verwendet wird, welche 2 bis 5 % des Gewichts der Endzusammensetzung darstellt.

19. Verfahren zur Herstellung einer pharmazeutischen Zusammensetzung mit modifizierter Wirkstofffreisetzung nach Anspruch 18, **dadurch gekennzeichnet, daß** der fetthaltige Matrixstoff ein Behensäure- und Alkoholester ist.

20. Verfahren zur Herstellung einer pharmazeutischen Zusammensetzung mit modifizierter Wirkstofffreisetzung nach einem der Ansprüche 1 bis 19, **dadurch gekennzeichnet, daß** ein Adjuvans benutzt wird, das unter den hydrophoben Verdünnungsmitteln, den hydrophilen Verdünnungsmitteln, den Bindemitteln und den Trennmitteln, einzeln oder als Gemisch ausgewählt wird.

21. Verfahren zur Herstellung einer pharmazeutischen Zusammensetzung mit modifizierter Wirkstofffreisetzung nach einem der Ansprüche 1 bis 20, **dadurch gekennzeichnet, daß** das hydrophobe Verdünnungsmittel das Di-Kalzium-Phospat ist und dadurch daß das hydrophile Verdünnungsmittel Laktose ist.

22. Pharmazeutische Zusammensetzung mit modifizierter Wirkstofffreisetzung, welche durch das Verfahren nach einem der Ansprüche 1 bis 21 hergestellt ist.
